# EUROPEAN PATENT SPECIFICATION

(11) **EP 0 622 672 B1**
(45) Date of publication and mention of the grant of the patent: **11.02.1998**
(21) Application number: 94201097.6
(22) Date of filing: 21.04.1994
(51) Int. Cl.: G03C 7/36, C07C 309/76, C07C 255/30, C07D 213/74, C07D 239/42, C07D 257/04

(54) **Photographic colour couplers, methods of making them and photographic materials containing them**
Photographische Farbkuppler, Verfahren zu ihrer Herstellung und photographische Materialien, die diese Kuppler enthalten
Coupleur pour la photographie en couleurs, procédés pour leur préparation et matériaux photographiques les contenant

(30) Priority: 24.04.1993 GB 9308551
(43) Date of publication of application: 02.11.1994
(73) Proprietor: KODAK LIMITED, Harrow, Middlesex HA1 4TY (GB)
(72) Inventor: Allway, Philip Arthur, c/o Kodak Limited, Harrow, Middlesex, HA1 4TY (GB)
(74) Representative: Nunney, Ronald Frederick Adolphe

(56) References cited:
- EP-A- 0 369 297
- EP-A- 0 618 494
- EP-A- 0 618 495
- EP-A- 0 618 497
- WO-A-92/14189
- WO-A-93/07534

## Description

### Field of the Invention

This invention relates to photographic colour couplers and in particular to a class of magenta couplers.

### Background of the Invention

Colour couplers are known to belong to a number of classes, for example magenta dye-forming couplers can be pyrazolones, pyrazolotriazoles and pyrazolobenzimidazoles while yellow dye-forming couplers can be acetanilides.

### Problem to be Solved by the Invention

There is always a need for new classes of couplers that have advantages over those already known to the art.

### Summary of the Invention

According to the present invention there are provided photographic elements comprising a support, at least one photosensitive silver halide layer and in or adjacent said silver halide layer a colour coupler of one of the general formulae: 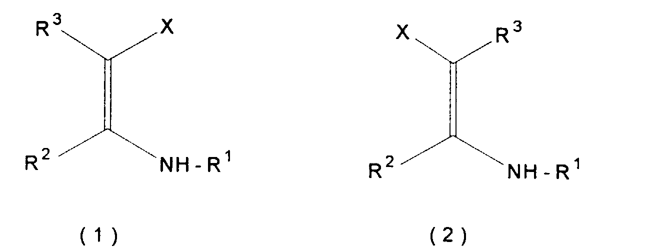 wherein
R¹ is a subtituted aryl or a subtituted heterocylic group, and
X is H or a coupling-off group,
characterised in that
R² is a substituted or unsubstituted, saturated or unsaturated, primary or secondary alkyl group in which the carbon atom which joins R² to the rest of the coupler has at least one fluorine atom attached to it,
R³ is an electron-withdrawing group,
and wherein the electron-withdrawing properties of R¹ are such that the dye formed on coupling with oxidised colour developing agent 4-(N-ethyl-N-2-hydroxyethyl)-2-methylphenylenediamine (CD4) has a λₘₐₓ of from 537 to 570 nm when measured in a coupler dispersion comprising 6% w/w gelatin, 8.8% coupler and coupler solvents in the ratio:- coupler : tricresyl phosphate : 2-(2-butoxyethoxy)ethyl acetate 1.0 : 0.5 : 1.5 (w/w) and in which the auxiliary solvent (2-(2-butoxyethoxy)ethyl acetate) is removed by washing the dispersion for 6 hours at 4°C and pH 6.0.

### Advantageous Effect of the Invention

The present invention provides a new class of couplers capable of forming magenta dyes having desirable spectral characteristics having maximum wavelength (λₘₐₓ) in the range from 537 to 570 nm.

### Cross-reference to Related Applications

The present couplers fall within the broad scope of the couplers claimed in our copending PCT Application No. EP92/02293. The methods of synthesis described therein are not suitable for preparing the compounds of the present invention.

### Detailed Description of the Invention

Examples of electron-withdrawing substituent groups for R¹ are listed below under R³. Particularly preferred electron-withdrawing substituents for R¹ are cyano, nitro, alkylsulphonyl and arylsulphonyl.

Examples of groups which R² may represent are trifluoromethyl, nonafluorobutyl and trifluoroethylene groups.

R³ may be an electron withdrawing group wherein the value of the Hammett substituent constant σₚ (SIGMAₚ as defined by Hansch et al, J. Med. Chem.,1973, 16, 1207; and ibid. 1977, 20, 304) is 0.03 or greater, preferably 0.35 or greater and more preferably 0.5 or above.

A substituent or atom wherein the value of the σₚ (SIGMAₚ) is 0.03 or above includes a fluorine atom, a chlorine atom, a bromine atom, an iodine atom, a substituted alkyl group (eg. trichloromethyl, trifluoromethyl, chloromethyl and perfluorobutyl), a nitrile group, an acyl group (eg. formyl, acetyl and benzoyl), a carboxyl group, a substituted or unsubstituted carbamoyl group (eg. methylcarbamoyl) an aromatic group substituted by another electron attractive group (eg pentachlorophenyl, pentafluorophenyl), a heterocyclic group which may be substituted (eg. 2-thienyl, 2-benzoxazolyl, 2-benzthiazolyl, 1-tetrazolyl and 1-phenyl-2-benzimidazolyl), a nitro group, an azo group (eg. phenylazo), an amino group substituted by another electron attractive group (eg. ditrifluoromethylamino), an alkoxy group substituted by another electron attractive group (eg. trifluoromethoxy), an alkylsulphonyloxy group (eg. methanesulphonyloxy), an acyloxy group (eg. acetyloxy, benzoyloxy), an arylsulphonyloxy group (eg. benzenesulphonyloxy), a phosphoryl group (eg. dimethoxyphosphoryl and diphenylphosphoryl), a thioalkyl group substituted by another electron attractive group (eg. trifluoromethyl), a sulphamoyl group, a sulphonamide group, a sulphonyl group (eg. methanesulphonyl, benzenesulphonyl), a thiocyanate, sulphoxide, carbonamido, alkyloxycarbonyl, aryloxycarbonyl, alkylsulphoxyl, arylsulphoxyl, sulphonamido, sulphonato group or a substituted phosphorus atom.

Examples of electron-withdrawing groups which R³ may represent are halogen, -CN, -NO₂, -OR⁴, -SR⁴, -SO₂R⁵, -OSO₂R⁵, -SOR⁵, -NHCOR⁵, -CONHR⁵_{,} -OCONHR⁵, -NHCO-OR⁵, -SO₂NH-R⁵, -NHSO₂R⁵, -NHSO₂NHR⁵, -NHNH-SO₂-R⁵, -COOH, -COOR⁵, -O-COR⁵, -COR⁵, -CSR⁵, -CONHNHR⁵, -CF₃, -NHR⁵, -NHR⁵R^{5'}, silyloxy, aryl, aralkyl, alkyl, cycloalkyl, ureido, imido, or a heterocycle,
wherein
R⁵ is an alkyl, cycloalkyl, aryl or heterocyclic group any of which may be substituted,
R^{5'} has the same definition as R⁵ and may be the same or different to R⁵, and
R⁴ is an alkyl, cycloalkyl, aryl or heterocyclic group any of which are optionally substituted,
and wherein the nature of the groups R⁵, R^{5'} and R⁴ and the substituents thereon are such that the group is electron-withdrawing.

Coupling-off groups (X) are well known in the art. Such groups can determine the equivalency of the coupler, i.e., whether it is a 2-equivalent or a 4-equivalent coupler, or modify the reactivity of the coupler. Such groups can advantageously affect the layer in which the coupler is coated, or other layers in the photographic recording material, by performing, after release from the coupler, functions such as dye formation, development acceleration or inhibition, bleach acceleration or inhibition, electron transfer facilitation, color correction and the like.

Examples of coupling-off groups include chloro, alkoxy, aryloxy (eg phenyloxy or 2-chloro-, 2,3,5-isopropyl-, 4-carboxy-, 4-carboxy-2-methylcarbonamido, 4-nitro-2-carbamoyl-phenyloxy), alkylthio, arylthio (eg phenylthio, or 2,3,5-isopropyl-phenylthio), heteroyloxy (eg pyridyloxy), isopropyl-phenylthio), heteroyloxy (eg pyridyloxy), sulfonyloxy, acyloxy, heterocyclyl joined via a ring carbon or hetero atom in the heterocyclic nucleus, sulfonamido, mercaptotetrazole, mercaptopropionic acid, phosphonyloxy and arylazo (eg 4-hydroxy-, 4-hexadecyloxy-3-methoxy-, 4-methyl-2-hydroxy-, 4-methylsulphonyl- or 4-t-butylcarbonamido-phenylazo).

Examples of coupling-off groups containing photographically useful groups (PUG's) are listed in Table 1 below.

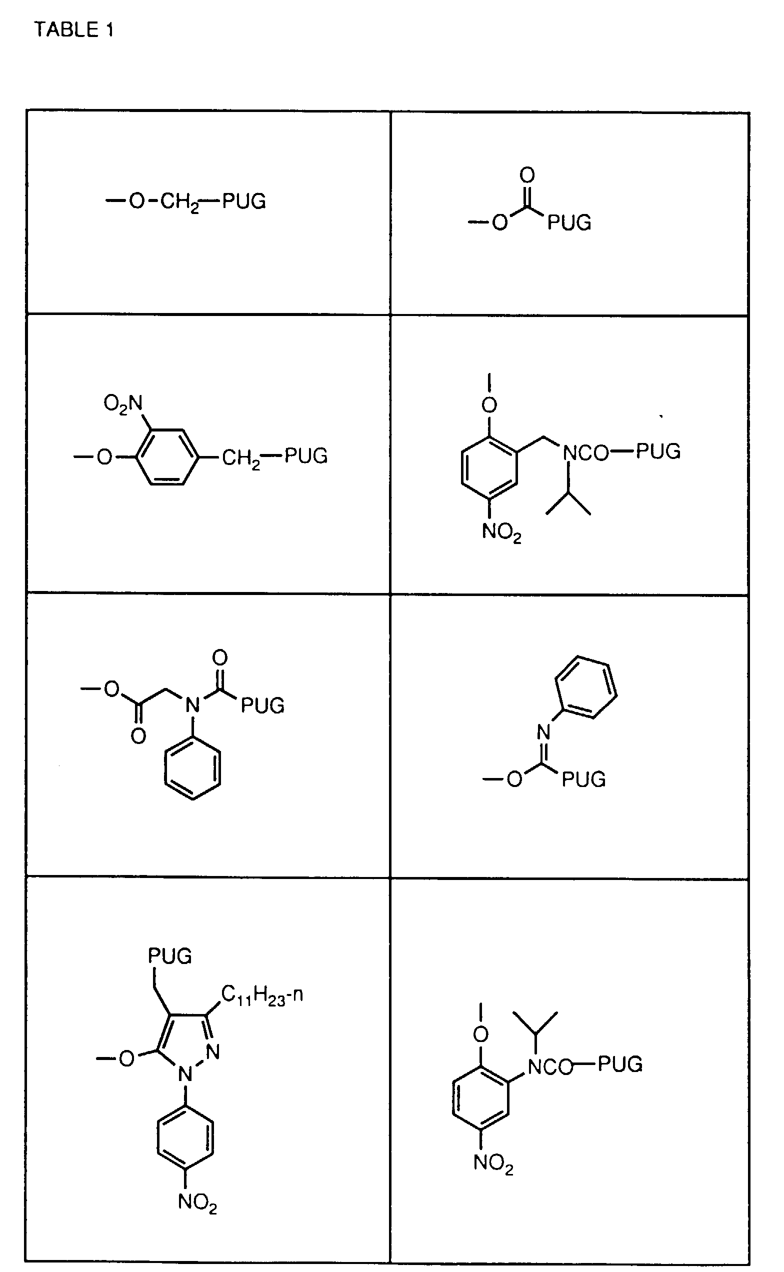

The present couplers may contain a ballast group of such size and configuration that the coupler is rendered non-diffusible in photographic layers. Such a ballast may form part of R¹, R² or R³.

The present invention further provides a photographic element comprising a support, at least one photosensitive silver halide layer and associated therewith a colour coupler of the present invention.

The present invention also provides a method by which the present couplers may be prepared in which the starting compound: 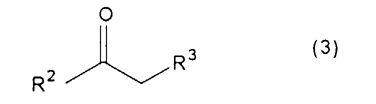 is reacted with a compound which is capable of combining with the oxygen of the starting compound to form a leaving group thus forming a compound of the formula (1) or (2) above wherein X is H. In one embodiment the reaction scheme is as follows: 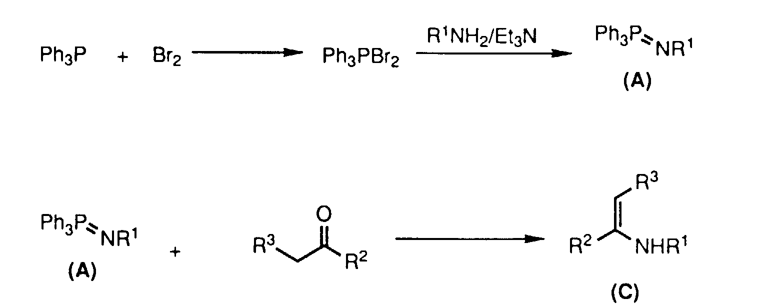 In another embodiment the reaction scheme is as follows:

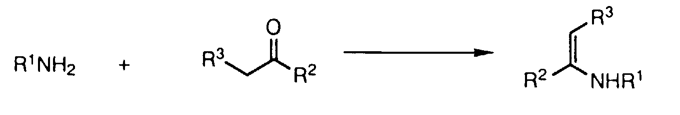

When it is desired to introduce a coupling-off group, the compound in which X is H is reacted with a compound which is a source of the coupling-off group as an electrophile or the starting compound (3) in the scheme above has the carbon between the carbon atom of the carbonyl group and R³ substituted with the coupling-off group.

Specific examples of couplers according to the present invention are listed below in Table 2.

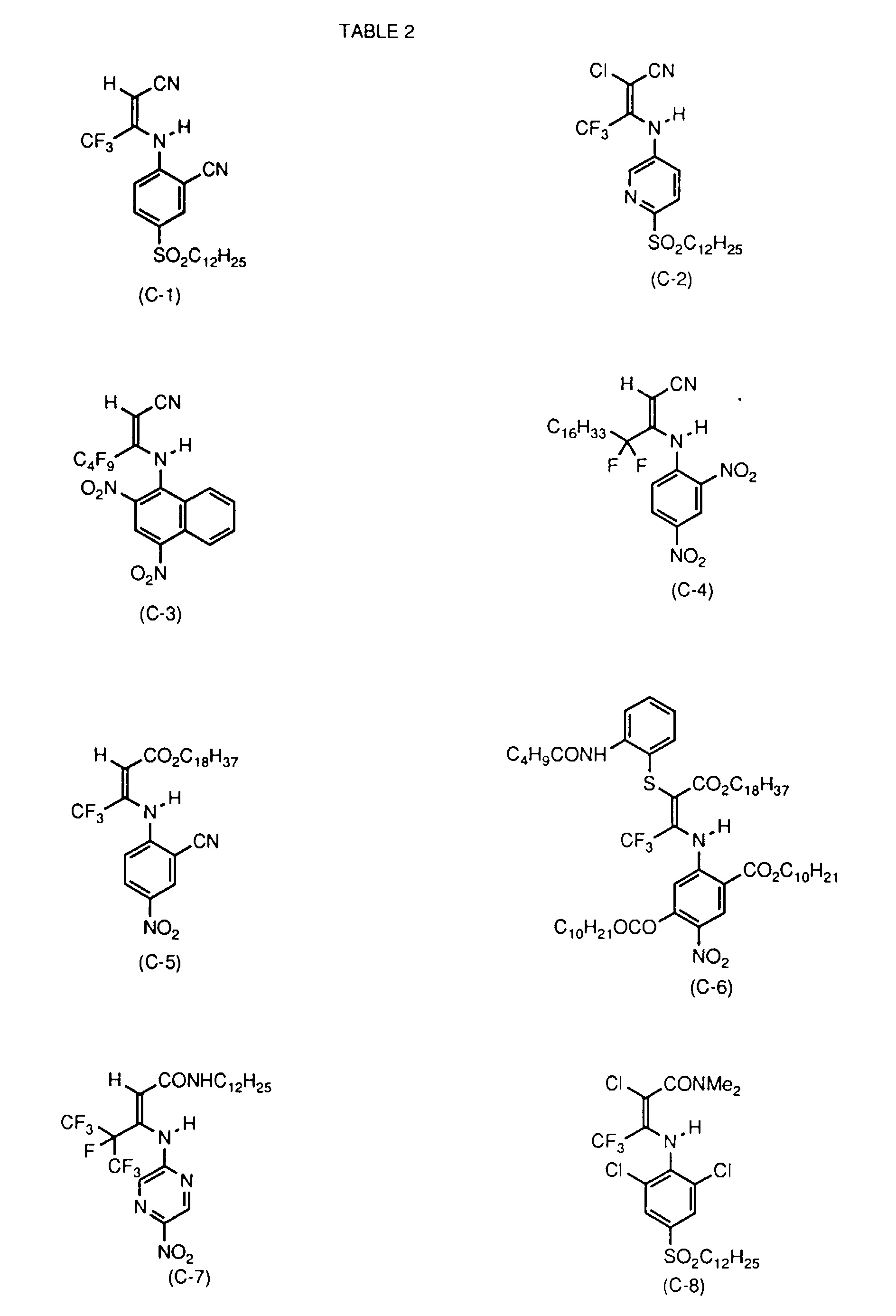

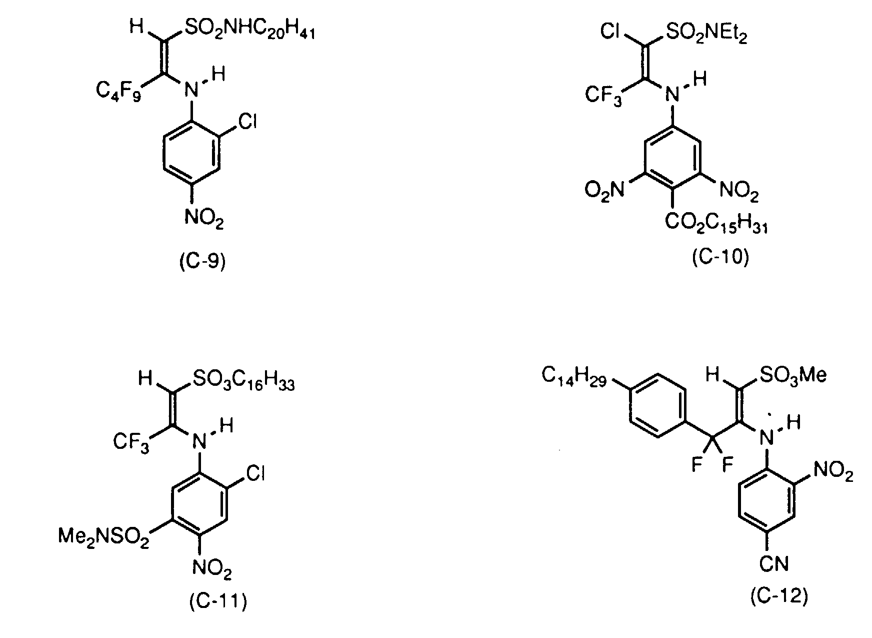

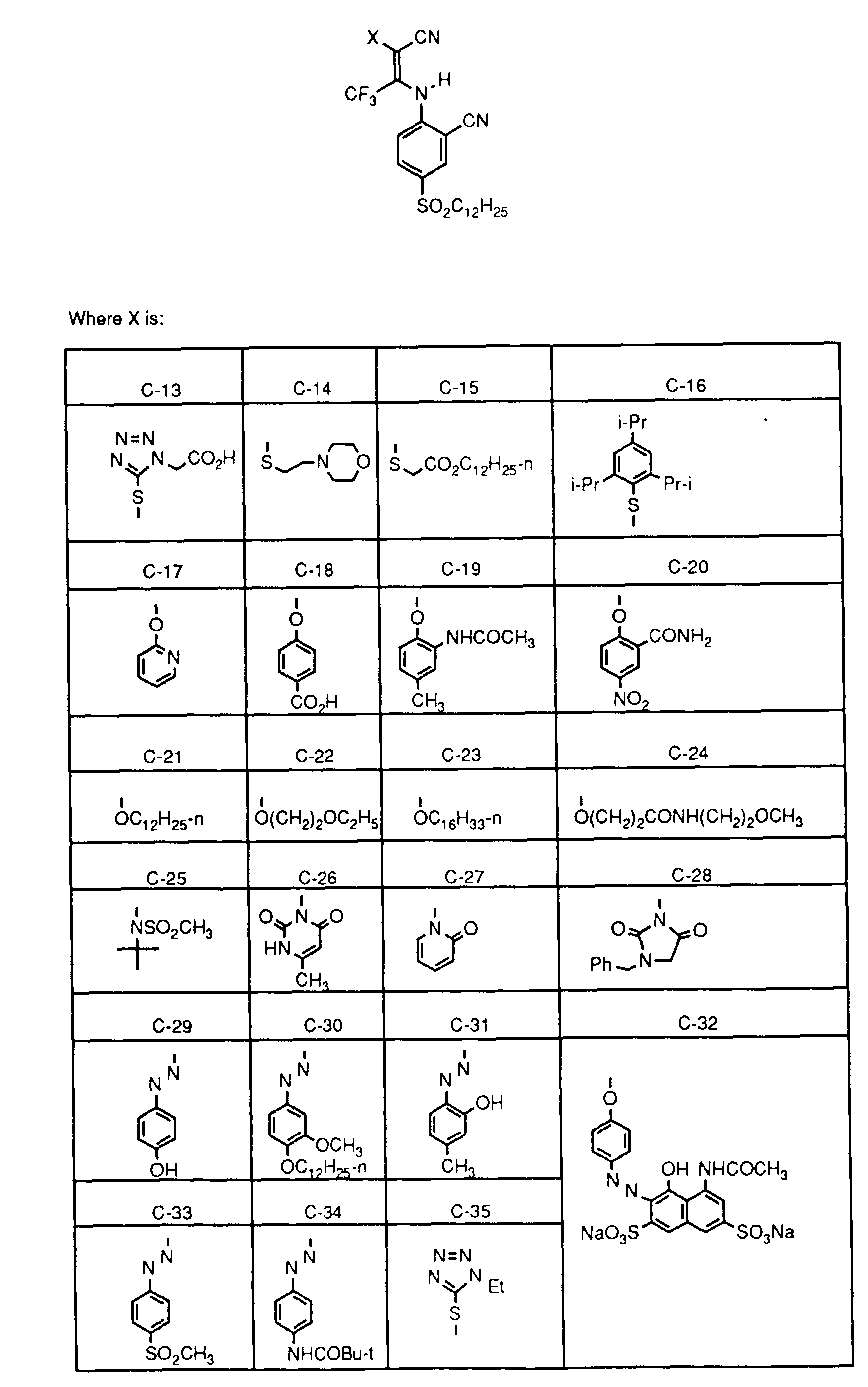

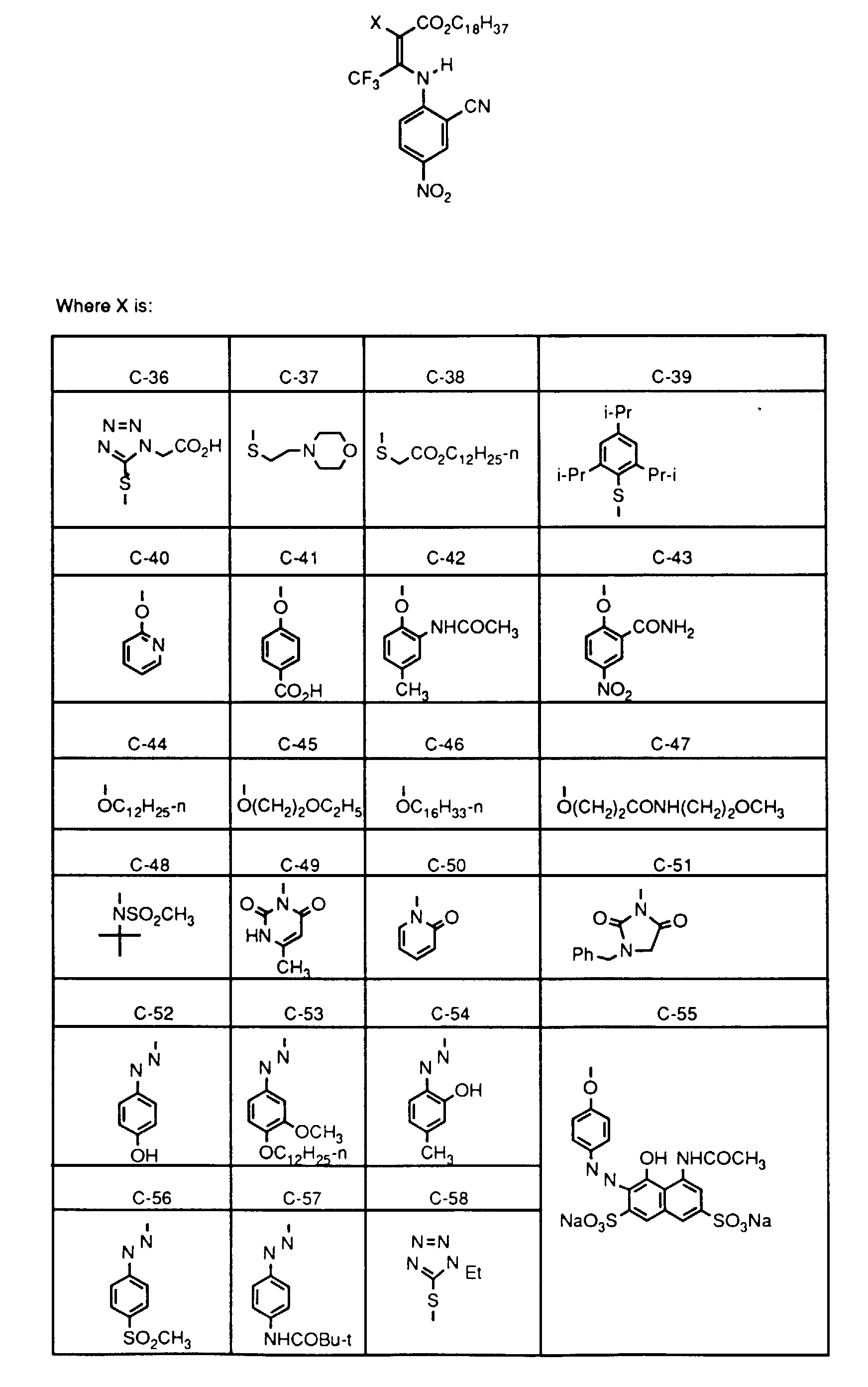

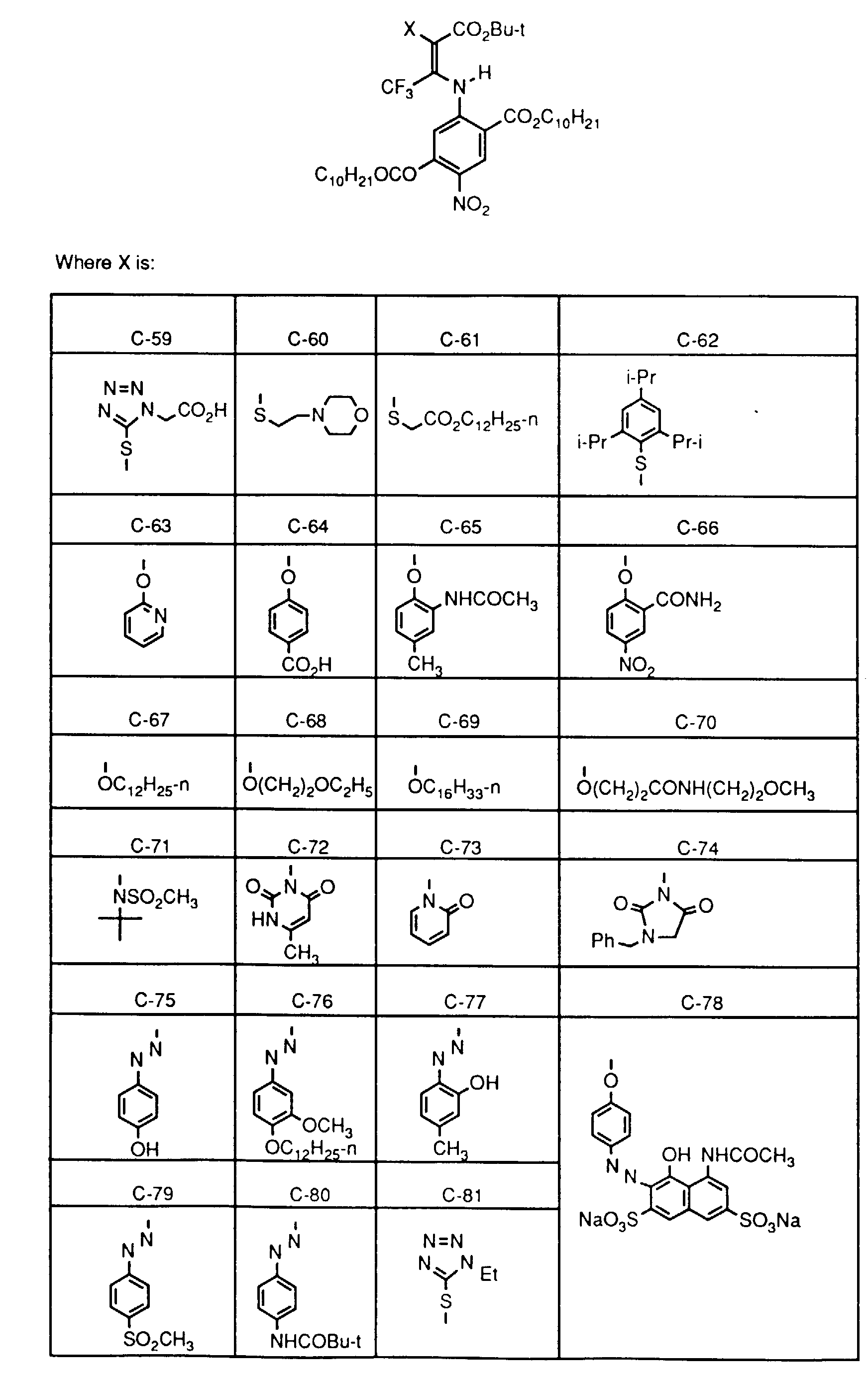

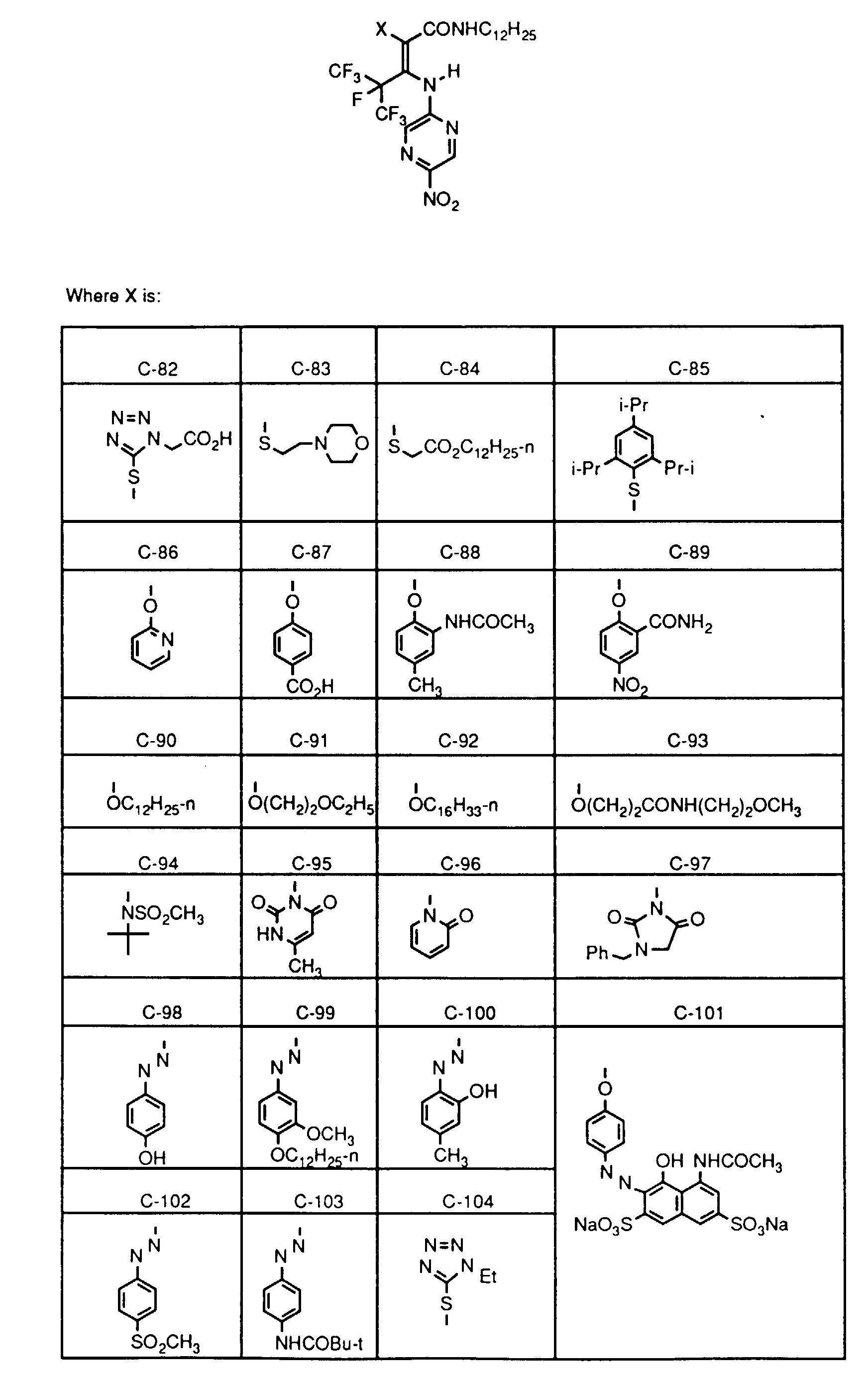

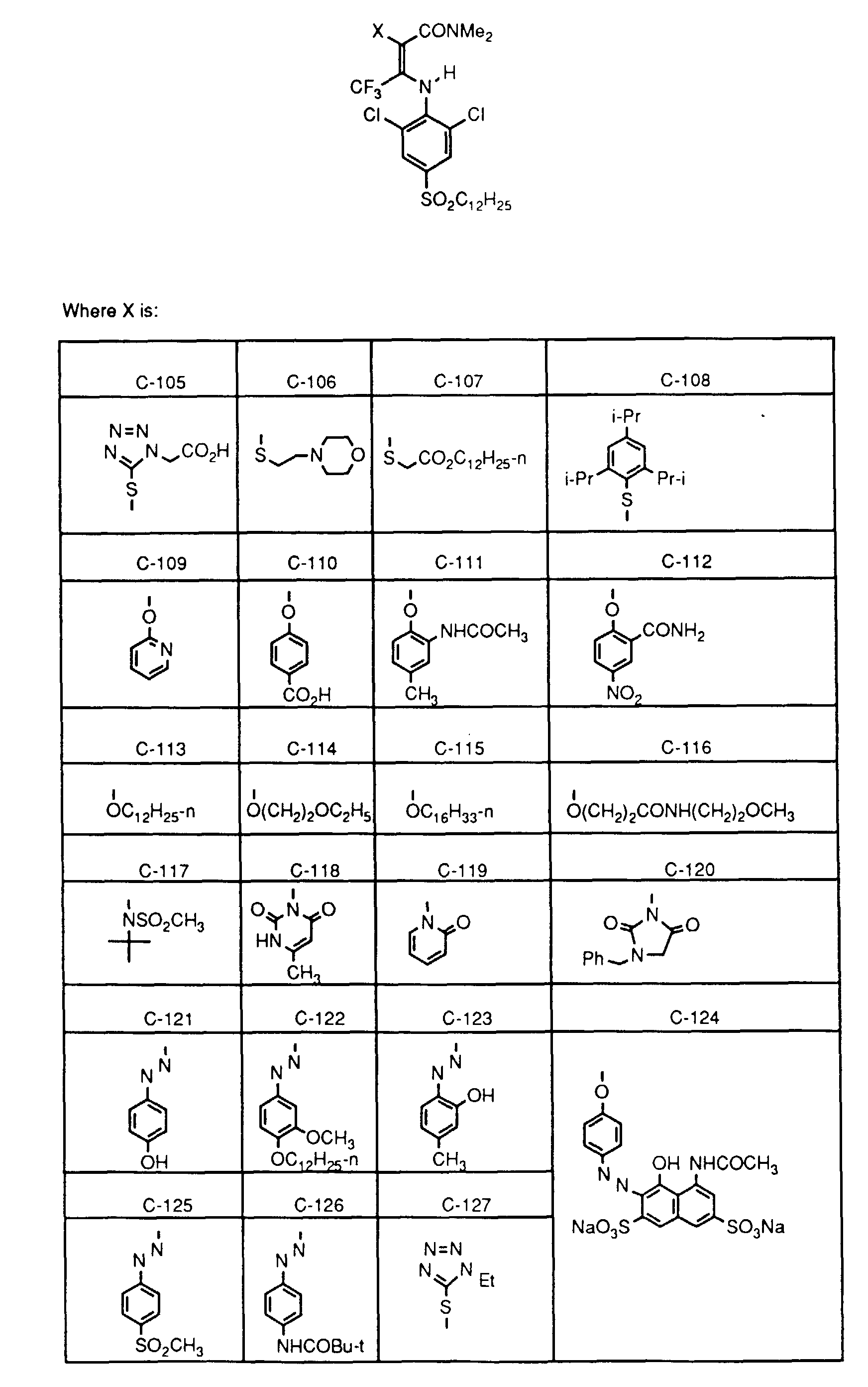

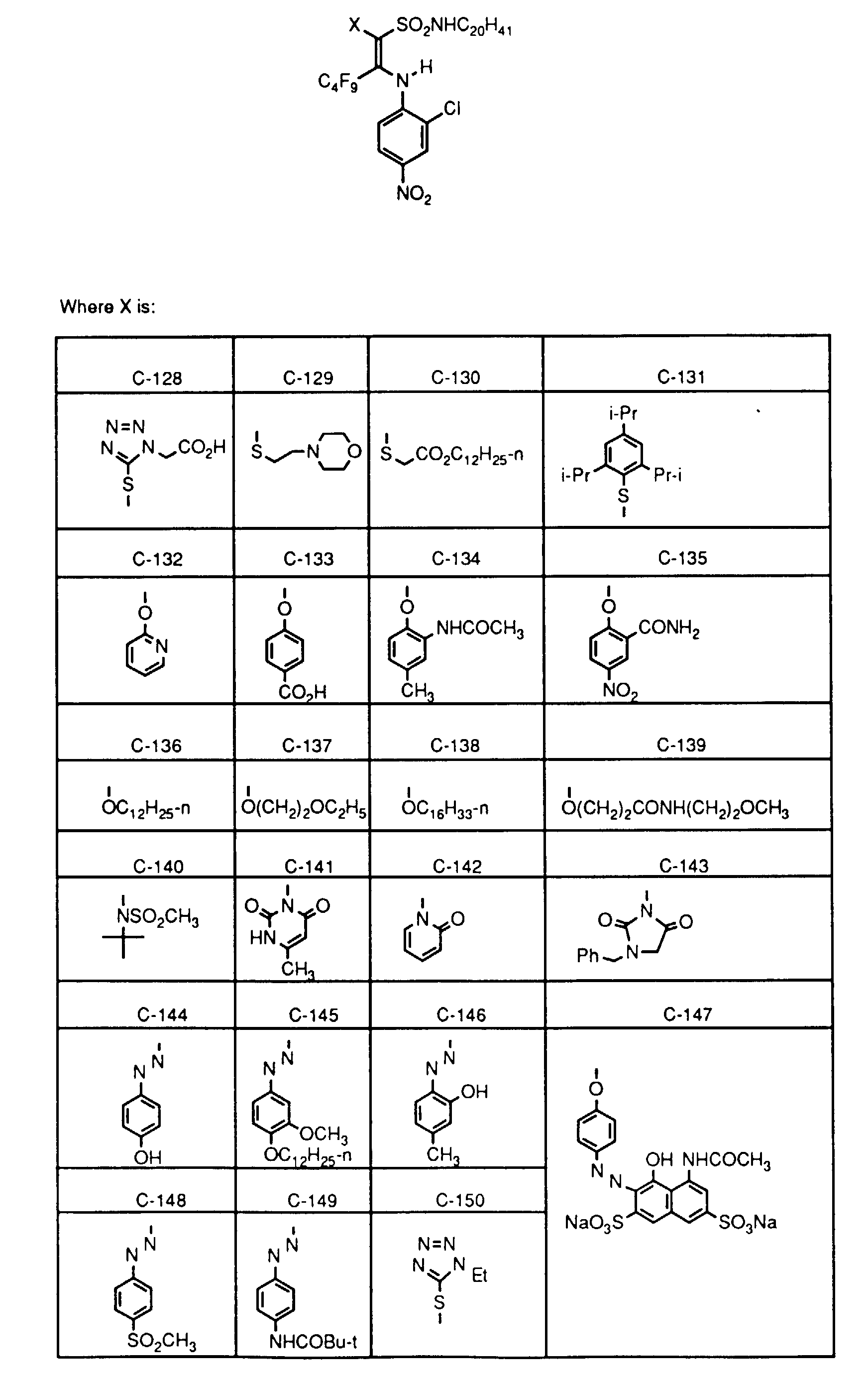

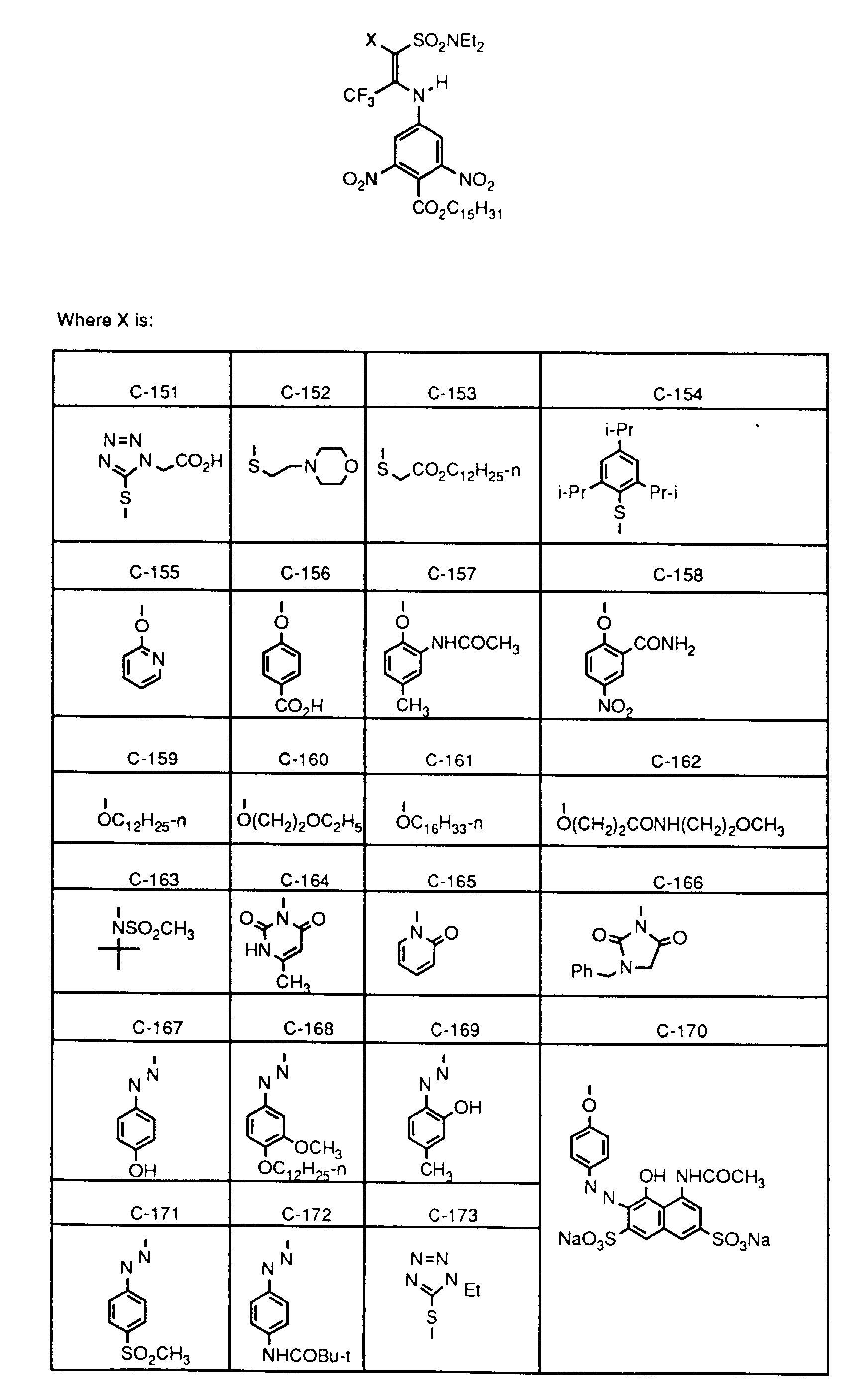

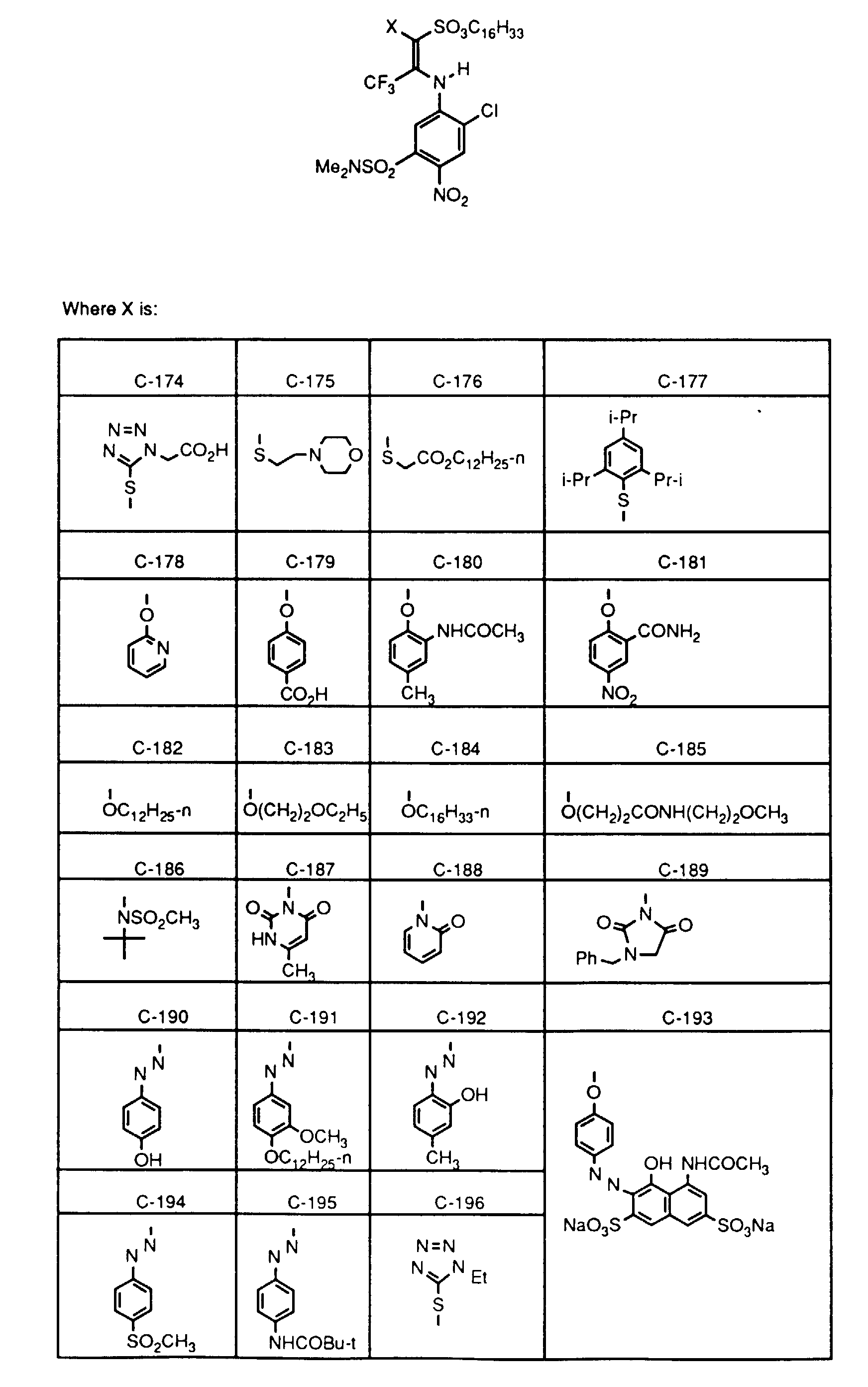

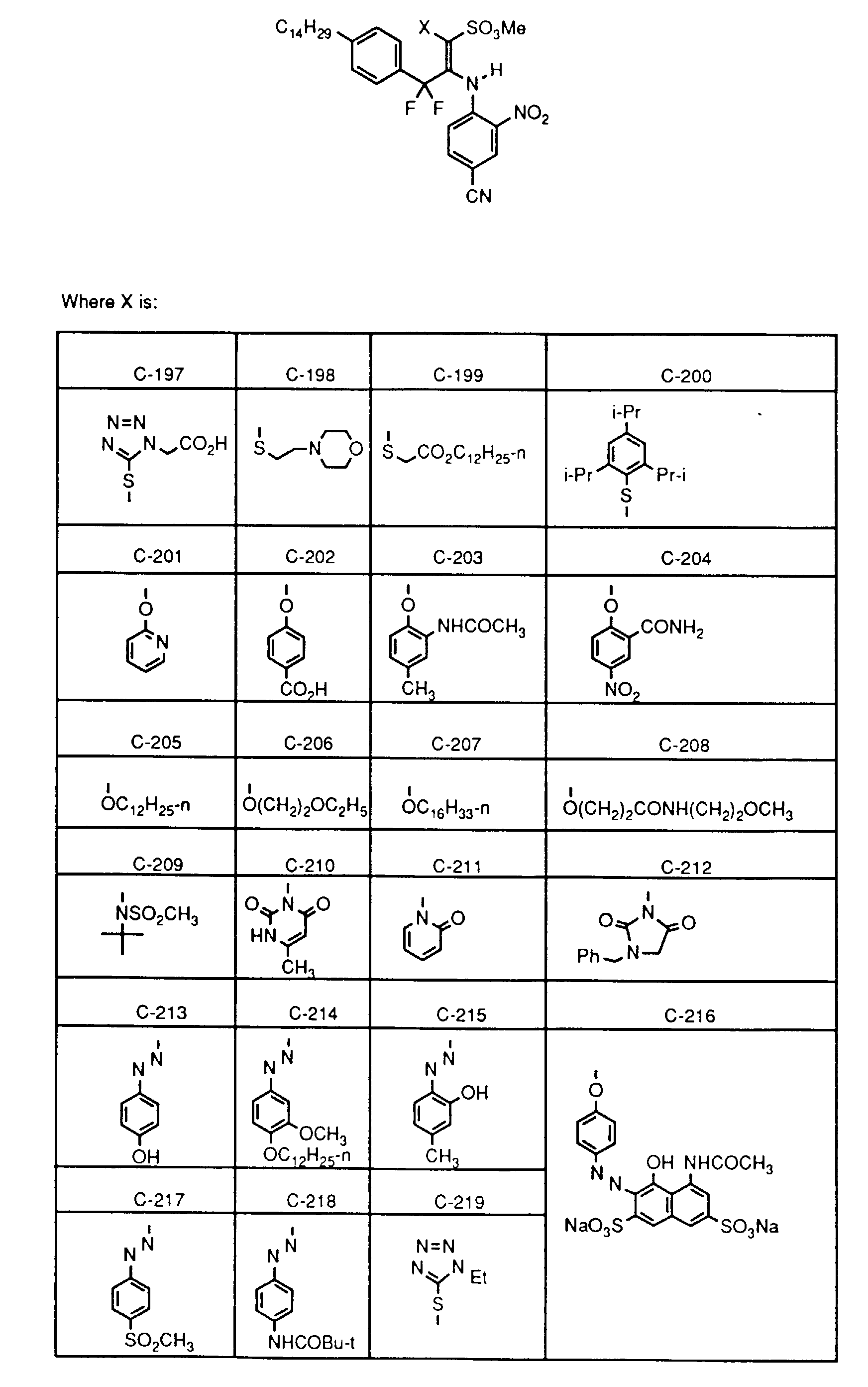

The dye-forming couplers of this invention can be used in the ways and for the purposes that dye-forming couplers have been previously used in the photographic art.

Typically, the couplers are associated with a silver halide emulsion layer coated on a support to form a photographic element. As used herein, the term "associated with" signifies that the coupler is incorporated in the silver halide emulsion layer or in a layer adjacent thereto where, during processing, it is capable of reacting with silver halide development products.

The photographic elements can be single colour elements or multicolour elements. In a multicolour element, the magenta dye-forming couplers of this invention would usually be associated with a green-sensitive emulsion, although they could be associated with an emulsion sensitised to a different region of the spectrum, or with a panchromatically sensitised, orthochromatically sensitised or unsensitised emulsion. Multicolour elements contain dye image-forming units sensitive to each of the three primary regions of the spectrum. Each unit can be comprised of a single emulsion layer or of multiple emulsion layers sensitive to a given region of the spectrum. The layers of the element, including the layers of the image-forming units, can be arranged in various orders as known in the art.

A typical multicolour photographic element comprises a support bearing yellow, magenta and cyan dye image-forming units comprising at least one blue-, green- or red-sensitive silver halide emulsion layer having associated therewith at least one yellow, magenta or cyan dye-forming coupler respectively, at least one of the magenta dye-forming couplers being a coupler of this invention. The element can contain additional layers, such as filter and barrier layers.

In the following discussion of suitable materials for use in the emulsions and elements of this invention, reference will be made to Research Disclosure Item 308119, December 1989 published by Kenneth Mason Publications, Emsworth, Hants, United Kingdom. This publication will be identified hereafter as "Research Disclosure".

The silver halide emulsion employed in the elements of this invention can be either negative-working or positive-working. Suitable emulsions and their preparation are described in Research Disclosure Sections I and II and the publications cited therein. Suitable vehicles for the emulsion layers and other layers of elements of this invention are described in Research Disclosure Section IX and the publications cited therein.

The elements of the invention can include additional couplers as described in Research Disclosure Section VII, paragraphs F G and H and the publications cited therein. The couplers of this invention and any additional couplers can be incorporated in the elements and emulsions as described in Research Disclosures of Section VII, paragraph C and the publications cited therein.

The photographic elements of this invention or individual layers thereof, can contain brighteners (see Research Disclosure Section V), antifoggants and stabilisers (see Research Disclosure Section VI), antistain agents and image dye stabiliser (see Research Disclosure Section VII, paragraphs I and J), light absorbing and scattering materials (see Research Disclosure Section VIII), hardeners (see Research Disclosure Section X), plasticisers and lubricants (see Research Disclosure Section XII), antistatic agents (see Research Disclosure Section XIII), matting agents (see Research Disclosure Section XVI) and development modifiers (see Research Disclosure Section XXI).

The photographic elements can be coated on a variety of supports as described in Research Disclosure Section XVII and the references described therein.

Photographic elements can be exposed to actinic radiation, typically in the visible region of the spectrum, to form a latent image as described in Research Disclosure Section XVIII and then processed to form a visible dye image as described in Research Disclosure Section XIX. Processing to form a visible dye image includes the step of contacting the element with a colour developing agent to reduce developable silver halide and oxidise the colour developing agent. Oxidised colour developing agent in turn reacts with the coupler to yield a dye.

Preferred colour developing agents are p-phenylene diamines. Especially preferred are 4-amino-3-methyl-N,N-diethylaniline hydrochloride, 4-amino-3-methyl-N-ethyl-N-β-(methanesulphonamido)-ethylaniline sulphate hydrate, 4-amino-3-methyl-N-ethyl-N-β-hydroxyethylaniline sulphate, 4-amino-3-β-(methanesulphonamido)ethyl-N,N-diethylaniline hydrochloride and 4-amino-N-ethyl-N-(2-methoxyethyl)-*m*-toluidine di-*p*-toluene sulphonate.

With negative-working silver halide emulsions this processing step leads to a negative image. To obtain a positive (or reversal) image, this step can be preceded by development with a non-chromogenic developing agent to develop exposed silver halide, but not form dye, and then uniform fogging of the element to render unexposed silver halide developable. Alternatively, a direct positive emulsion can be employed to obtain a positive image.

Development is followed by the conventional steps of bleaching, fixing, or bleach-fixing, to remove silver and silver halide, washing and drying.

The following Examples are included for a better understanding of the invention.

### EXAMPLE 1

### Preparation of 2-(N-(2-Cyano-4-(dodecylsulphonyl)-anilino))-2-trifluoromethylacrylonitrile

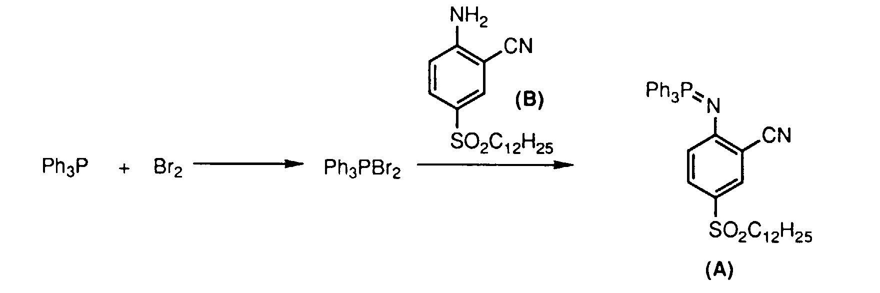

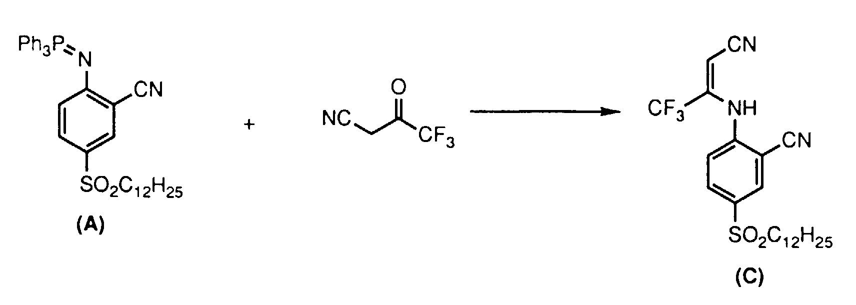

### N-(2-Cyano-4-(dodecanesulphonyl)phenyl)triphenyl-phosphine imine (A)

Bromine (0.62g, 3.88mmol) in 1,2-dichloroethane (3ml) was added dropwise to stirred solution of triphenylphoshine (1.01g, 3.85mmol) in 1,2-dichloroethane (30ml) under a nitrogen atmosphere. The temperature of the reaction was maintained at less than 5°C by the use of an ice/water bath. After the addition was complete a mixture of the aniline **B** (0.90g, 2.57mmol) and triethylamine (0.78g, 7.72mmol) in warm 1,2-dichloroethane (25ml) was added in one portion to the suspension and the mixture heated to reflux. After being heated at reflux for 1 hour the solution was allowed to cool to room temperature and the solid was filtered-off. Concentration of the filtrate in vacuo gave an oil which crystallised upon standing. This solid was suspended in hot ethyl acetate, filtered and allowed to cool to give the product (0.95g, 1.56mmol, 61%) as a white crystalline solid. All spectroscopic data is consistent with the proposed structure.

### 2-(N-(2-Cyano-4-(dodecylsulphonyl)-anilino))-2-trifluoromethylacrylonitrile (C)

A mixture of the phosphine imine **A** (0.80g, 1.31mmol) and 3-cyano-1,1,1-trifluoroacetone (0.23g, 1.68mmol) was heated rapidly to reflux in 1,2-dichloroethane (10ml). After 2 hours the slightly cloudy solution was concentrated in vacuo to give a yellow oil (1.07g) which was purified by column chromatography over silica (eluent 2:1 60-80 petroleum ether/ethyl acetate) to give the product (0.42g, 0.896mmol, 68%) as a white solid. All spectroscopic data is consistent with the proposed structure.

### EXAMPLE 2

### Preparation of 2-(N-(3-nitroanilino))-2-trifluoromethylacrylonitrile

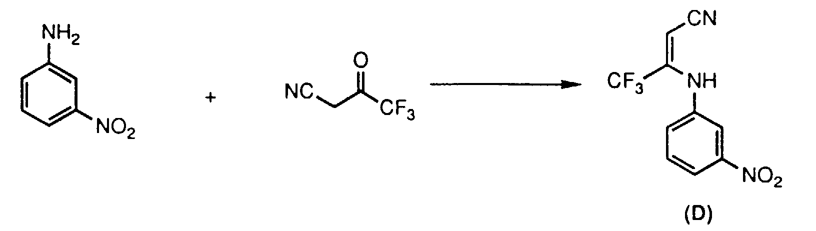

### 2-(N-(3-nitroanilino))-2-trifluoromethylacrylonitrile (D)

A mixture of m-nitroaniline (0.94g, 6.81mmol), 3-cyano-1,1,1-trifluoroacetone (0.93g, 6.79mmol and p-toluenesulphonic acid (catalytic amount) in toluene (10ml) was heated at reflux, slowly allowing the toluene to distill off. After 2 hours the reaction was allowed to cool and then concentrated in vacuo to give a brown oily-solid. This was purified by column chromatography over silica (eluent 3:1 60-80 petroleum ether/ethyl acetate) to give the product (0.112g, 0.436mmol, 6%) as a yellow oil which crystallised upon standing. All spectroscopic data is consistent with the proposed structure.

### EXAMPLE 3

Compound C-1 of the present invention and control compound A were incorporated into a photographic silver bromoiodide emulsion and coated in the following format:-

| | | |
|---|---|---|
| Gel Supercoat | | |
| Gelatin | | 1.50 gm/m² |
| Emulsion Layer | | |
| Silver bromoiodide | | 1.61 gm/m² |
| Coupler | | 1.04 mmol/m² |
| Gelatin | | 2.42 gm/m² |
| Bis(vinylsulphonyl)methane (hardener) | | 0.06 gm/m² |

| /// Support - Cellulose acetate //// | | |
|---|---|---|
| | | |

Control compound A had the following formula:

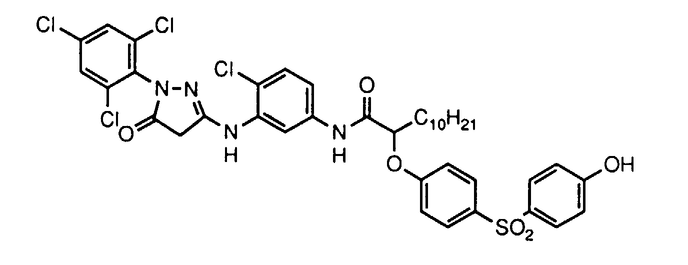

The coupler dispersion used contained 6% w/w gelatin, 8.8% coupler and coupler solvents in the ratio:- coupler : tricresyl phosphate : 2-(2-butoxyethoxy)ethyl acetate 1.0 : 0.5 : 1.5 (w/w). The auxiliary solvent (2-(2-butoxyethoxy)ethyl acetate) was removed by washing the dispersion for 6 hours at 4° C and pH 6.0.

### Spectrophotometric testing

The experimental photographic coatings prepared in this way were slit and chopped into 30cm x 35mm test strips. These strips were exposed (0.1 sec) through a 0-0.9ND step-wedge (0.3ND steps) test object and Daylight V, Wratten 9 filters and the correct ND filters to give an optical density of about 1.0. The strips were processed through a C-41 process as described in the British Journal of Photography (1988) 196-198 using the following process times:

| | |
|---|---|
| Developer | 2.5 minutes |
| Bleach | 4.0 minutes |
| Wash | 2.0 minutes |
| Fix | 4.0 minutes |
| Wash | 2.0 minutes |

and samples cut from the magenta dye image step with density closest to 1.0. Visible absorption spectra (normalised to 1.0 density) were obtained using a PyeUnicam SP8-100 spectrophotometer. Dye hues are expressed in terms of the wavelength of maximium absorption (λₘₐₓ).

### Results

| **Coupler** | λ_{**max**}**/nm** |
|---|---|
| A | 546.0 |
| C-1 | 548.5 |

Coupler C-1 had very similar spectral absorption properties as known coupler A.

## Claims

1. A photographic element comprising a support, at least one photosensitive silver halide layer and in or adjacent said silver halide layer a colour coupler of one of the general formulae: 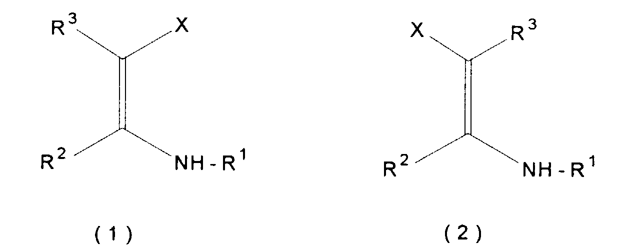 wherein
R¹ is a substituted aryl or a substituted heterocylic group, R¹ being substituted with an electron-withdrawing substitutent,
X is H or a coupling-off group,
characterised in that
R² is a substituted or unsubstituted, saturated or unsaturated, primary or secondary alkyl group in which the carbon atom which joins R² to rest of the coupler has at least one fluorine atom attached to it,
R³ is an electron-withdrawing group,
and wherein the electron-withdrawing properties of R¹ are such that the dye formed on coupling with oxidised colour developing agent 4-(N-ethyl-N-2-hydroxyethyl)-2-methylphenylenediamine (CD4) has a λₘₐₓ of from 537 to 570 nm when measured in a coupler dispersion comprising 6% w/w gelatin, 8.8% coupler and coupler solvents in the ratio:- coupler : tricresyl phosphate : 2-(2-butoxyethoxy)ethyl acetate 1.0 : 0.5 : 1.5 (w/w) and in which the auxiliary solvent (2-(2-butoxyethoxy)ethyl acetate) is removed by washing the dispersion for 6 hours at 4°C and pH 6.0.

2. A photographic element as claimed in claim 1 in which R¹ is substituted with a cyano, nitro, alkylsulphonyl and/or arylsulphonyl group.

3. A photographic element as claimed in any of claims 1 to 2 in which R² is a trifluoromethyl, nonafluorobutyl or trifluoroethylene group.

4. A photographic element as claimed in any of claims 1 to 3 in which R³ is halogen, -CN, -NO₂, -OR⁴, -SR⁴, -SO₂R⁵, -OSO₂R⁵, -SOR⁵, -NHCOR⁵, -CONHR⁵_{,} - OCONHR⁵, -NHCO-OR⁵, -SO₂NH-R⁵, -NHSO₂R⁵, -NHSO₂NHR⁵, -NHNH-SO₂-R⁵, -COOH, -COOR⁵, -O-COR⁵, -COR⁵, -CSR⁵, -CONHNHR⁵, -CF₃, -NHR⁵, -NHR⁵R^{5'}, silyloxy, aryl, aralkyl, alkyl, cycloalkyl, ureido, imido, or a heterocycle,
wherein
R⁵ is an alkyl, cycloalkyl, aryl or heterocyclic group any of which may be substituted,
R^{5'} has the same definition as R⁵ and may be the same or different to R⁵, and
R⁴ is an alkyl, cycloalkyl, aryl or heterocyclic group any of which are optionally substituted,
and wherein the nature of the groups R⁵, R^{5'} and R⁴ and the substituents thereon are such that the group is electron-withdrawing.

5. A photographic element as claimed in any of claims 1 to 4 in which X is hydrogen, chloro, alkoxy, phenyloxy, or 2-chloro-, 2,3,5-isopropyl-, 4-carboxy-, 4-carboxy-2-methyl-carbonamido-, or 4-nitro-2-carbamoyl-phenyloxy, alkylthio, phenylthio, or 2,3,5-isopropyl-phenylthio, pyridyloxy, sulfonyloxy, acyloxy, sulfonamido, mercaptotetrazolyl, mercaptopropionoyl, phosphonyloxy or 4-hydroxy-, 4-hexadecyloxy-3-methoxy- 4-methyl-2-hydroxy-, 4-methylsulphonyl- or 4-t-butylcarbonamido-phenylazo.

6. A magenta colour coupler as defined in any of claims 1 to 5.

7. A method for preparing couplers of the formula (1) or (2) of claim 1
(a) in which X is H in which the starting compound: 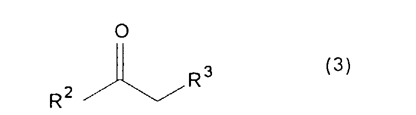 is reacted with a compound which is capable of combining with the oxygen of the starting compound to form a leaving group thus forming a compound of the formula (1) or (2) of claim 1 wherein X is H, or
(b) in which X is other than H in which the compound which is a source of the coupling-off group is an electrophile or the starting compound (3) has the carbon between the carbon atom of the carbonyl group and R³ is substituted with the coupling-off group.

8. A method as claimed in claim 7 which corresponds to one of the reaction schemes: 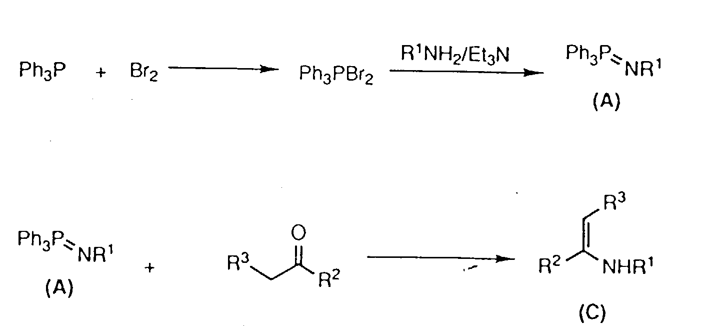 or: 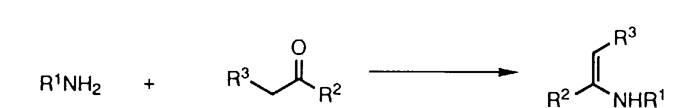 in which R¹, R² and R³ are as defined in claim 1.

## Patentansprüche

1. Photographisches Element mit einem Träger, mindestens einer photosensitiven Silberhalogenidschicht und in oder benachbart zur Silberhalogenidschicht einem Farbkuppler mit einer der allgemeinen Formeln: 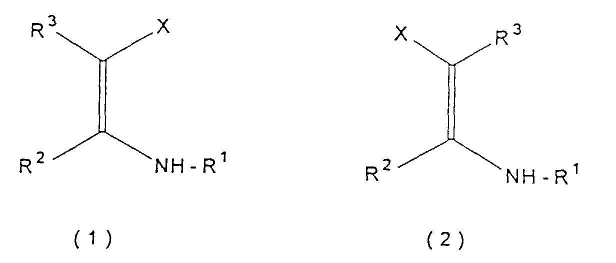 worin
R¹ eine substituierte Aryl- oder eine substituierte heterocyclische Gruppe ist, R¹ substituiert ist durch einen Elektronen abziehenden Substituenten,
X für H oder eine abkuppelnde Gruppe steht,
dadurch gekennzeichnet, daß R² eine substituierte oder unsubstituierte gesättigte oder ungesättigte,
primäre oder sekundäre Alkylgruppe ist, in der das Kohlenstoffatom, das R² an den Rest des Kupplers bindet, mindestens ein Fluoratom aufweist, das an dieses gebunden ist,
R³ eine Elektronen abziehende Gruppe ist,
und worin die Elektronen abziehenden Eigenschaften von R¹ derart sind, daß der durch Kupplung mit dem oxidierten Farbentwicklungsmittel 4-(N-Ethyl-N-2-hydroxyethyl)-2-methylphenylendiamin (CD4) erzeugte Farbstoff einen λₘₐₓ-Wert von 537 bis 570 nm aufweist, gemessen in einer Kupplerdispersion mit 6 % w/w Gelatine, 8,8 % Kuppler und Kupplerlösungsmittel im Verhältnis: Kuppler : Tricresylphosphat : 2-(2-Butoxyethoxy)ethylacetat 1,0 : 0,5 : 1,5 (w/w) und wobei das Hilfslösungsmittel (2-(2-Butoxyethoxy)ethylacetat) durch Waschen der Dispersion während 6 Stunden bei 4°C und einem pH-Wert von 6,0 entfernt wird.

2. Photographisches Element nach Anspruch 1, in dem R¹ substituiert ist durch eine Cyano-, Nitro-, Alkylsulphonyl- und/oder Arylsulphonylgruppe.

3. Photographisches Element nach einem der Ansprüche 1 und 2, worin R² steht für eine Trifluoromethyl-, Nonafluorobutyl- oder Trifluoroethylengruppe.

4. Photographisches Element nach einem der Ansprüche 1 bis 3, in dem R³ steht für Halogen, -CN, -NO₂, -OR⁴, SR⁴, -SO₂R⁵, -OSO₂R⁵, -SOR⁵, -NHCOR⁵, -CONHR⁵, -OCONHR⁵, -NHCO-OR⁵, -SO₂NH-R⁵, -NHSO₂R⁵, -NHSO₂NHR⁵, NHNH-SO₂-R⁵, -COOH, -COOR⁵, -O-COR⁵, -COR⁵, -CSR⁵, -CONHNHR⁵, -CF₃, -NHR⁵, -NHR⁵R^{5'}, Silyloxy, Aryl, Aralkyl, Alkyl, Cycloalkyl, Ureido, Imido oder eine heterocyclische Gruppe, worin
R⁵ seht für eine Alkyl-, Cycloalkyl-, Aryl- oder heterocyclische Gruppe, von denen eine jede substituiert sein kann,
R^{5'} die gleiche Definition wie R⁵ hat und gleich oder verschieden von R⁵ sein kann und worin
R⁴ eine Alkyl-, Cycloalkyl-, Aryl- oder heterocyclische Gruppe ist, von denen eine jede gegebenenfalls substituiert sein kann,
und worin die Natur der Gruppen R⁵, R^{5'} und R⁴ und hieran befindlichen Substituenten derart ist, daß die Gruppe elektronenabziehend ist.

5. Photographisches Element nach einem der Ansprüche 1 bis 4, in dem X steht für Wasserstoff, Chloro, Alkoxy, Phenyloxy, oder 2-Chloro-, 2,3,5-Isopropyl-, 4-Carboxy-, 4-Carboxy-2-methylcarbonamido-, oder 4-Nitro-2-carbamoyl-phenyloxy, Alkylthio, Phenylthio, oder 2,3,5-Isopropyl-phenylthio, Pyridyloxy, Sulfonyloxy, Acyloxy, Sulfonamido, Mercaptotetrazolyl, Mercaptopropionoyl, Phosphonyloxy oder 4-Hydroxy-, 4-Hexadecyloxy-3-methoxy-4-methyl-2-hydroxy-, 4-Methylsulphonyl- oder 4-t-Butylcarbonamido-phenylazo.

6. Purpurrotkuppler wie in Ansprüchen 1 bis 5 definiert.

7. Verfahren zur Herstellung von Kupplern der Formeln (1) oder (2) gemäß Anspruch 1
(a) wobei X für H steht, wobei die Ausgangsverbindung: 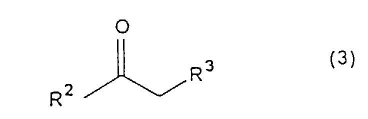 umgesetzt wird mit einer Verbindung, die dazu befähigt ist, sich mit dem Sauerstoff der Ausgangsverbindung zu kombinieren unter Erzeugung einer sich abspaltenden Gruppe unter Erzeugung einer Verbindung der Formel (1) oder (2) von Anspruch 1, worin X für H steht, oder
(b) worin X von H verschieden ist, wobei die Verbindung, die ein Lieferant der abkuppelnden Gruppe ist, ein Elektrophil ist, oder die Ausgangsverbindung (3) hat das Kohlenstoffatom zwischen dem Kohlenstoffatom der Carbonylgruppe und R³ ist durch die abkuppelnde Gruppe substituiert.

8. Verfahren nach Anspruch 7, das einem der Reaktionsschemen entspricht: 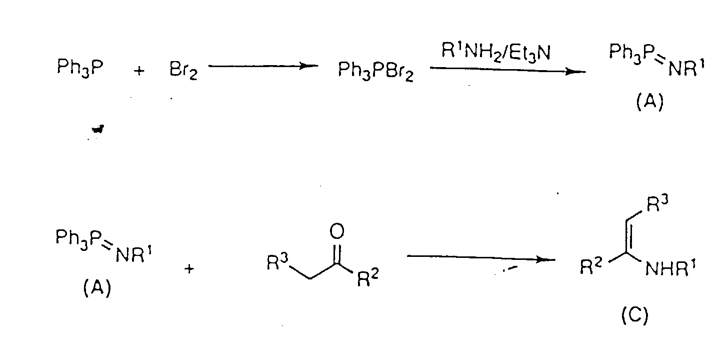 oder 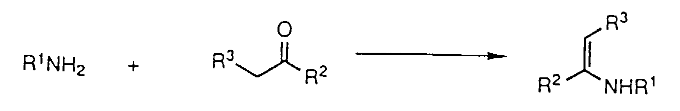 worin R¹, R² und R³ die in Anspruch 1 angegebene Bedeutung haben.

## Revendications

1. Elément photographique comprenant un support, au moins une couche photosensible aux halogénures d'argent et dans ladite couche aux halogénures d'argent ou dans une couche adjacente, un coupleur chromogène représenté par l'une des formules générales : 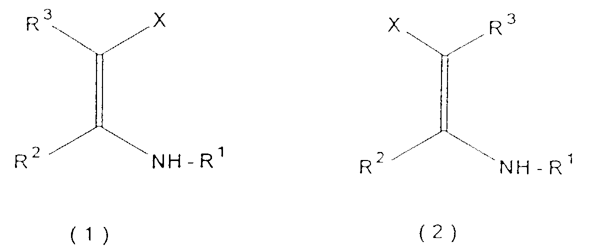 où
R¹ est un groupe aryle substitué ou un groupe hétérocyclique substitué, R¹ étant substitué par un substituant attracteur d'électrons,
X est H ou un groupe se séparant au couplage,
caractérisé en ce que
R² est un groupe alkyle primaire ou secondaire, substitué ou non, saturé ou non, dans lequel l'atome de carbone qui relie R² au reste du coupleur comprend au moins un atome de fluor auquel il est rattaché,
R³ est un groupe attracteur d'électrons, et dans lequel les propriétés attractrices d'électrons du groupe R¹ sont telles que le colorant formé au cours du couplage avec le développateur chromogène oxydé, la 4-(N-éthyl-N-2-hydroxyéthyl)-2-méthylphénylènediamine (CD4) a une λₘₐₓ de 537 à 570 nm, mesurée dans une dispersion de coupleurs comprenant 6 % en poids de gélatine, 8,8 % de coupleur et de solvants de coupleur selon le rapport suivant : - coupleur : phosphate de tricrésyle : acétate de 2-(2-butoxyéthoxy)éthyle : 1,0 : 0,5 : 1,5 (en poids) et dans lequel le solvant auxiliaire, l'acétate de (2-(2-butoxyéthoxy)éthyle est éliminé en lavant la dispersion pendant 6 heures à 4°C et à un pH de 6,0.

2. Elément photographique selon la revendication 1, dans lequel R¹ est substitué par un groupe cyano, nitro, alkylsulfonyle et/ou arylsulfonyle.

3. Elément photographique selon l'une quelconque des revendications 1 à 2, dans lequel R² est un groupe trifluorométhyle, nonafluorobutyle ou trifluoroéthylène.

4. Elément photographique selon l'une quelconque des revendications 1 à 3, dans lequel R³ est un halogène, -CN, -NO₂, -OR⁴, -SR⁴, -SO₂R⁵, -OSO₂R⁵, -SOR⁵, -NHCOR⁵, -CONHR⁵, -OCONHR⁵, -NHCO-OR⁵, -SO₂NH-R⁵, -NHSO₂R⁵, -NHSO₂NHR⁵, -NHNH-SO₂-R⁵, -COOH, -COOR⁵, -O-COR⁵, -COR⁵, -CSR⁵, -CONHNHR⁵, -CF₃, -NHR⁵, -NHR⁵R^{5'}, silyloxy, aryle, aralkyle, alkyle, cycloalkyle, uréido, imido ou un hétérocycle, où
R⁵ est un groupe alkyle, cycloalkyle, aryle ou un groupe hétérocyclique, l'un quelconque de ces groupes pouvant être substitué,
R^{5'} a la même définition que R⁵ et peut être identique ou différent de R⁵, et
R⁴ est un groupe alkyle, cycloalkyle, aryle ou hétérocyclique dont l'un quelconque peut être éventuellement substitué,
et où la nature des groupes R⁵, R^{5'} et R⁴ et les substituants de ces groupes sont tels que le groupe est attracteur d'électrons.

5. Elément photographique selon l'une quelconque des revendications 1 à 4, dans lequel X est l'hydrogène, chloro, alcoxy, phényloxy ou 2-chloro, 2,3,5-isopropyle, 4-carboxy-, 4-carboxy-2-méthylcarbonamido- ou 4-nitro-2-carbamoyl-phényloxy, alkylthio, phénylthio, ou 2,3,5-isopropyl-phénylthio, pyridyloxy, sulfonyloxy, acyloxy, sulfonamido, mercaptotétrazolyle, mercaptopropionyle, phosphonyloxy ou 4-hydroxy-, 4-hexadécyloxy-3-méthoxy-4-méthyl-2-hydroxy-, 4-méthylsulfonyl- ou 4-t-butylcarbonamidophénylazo.

6. Coupleur chromogène magenta tel que défini dans l'une quelconque des revendications 1 à 5.

7. Procédé de préparation des coupleurs de formule (1) ou (2) de la revendication 1
(a) dans lequel X est H, dans lequel le composé de départ : 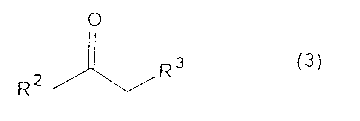 réagit avec un composé capable de se combiner avec l'oxygène du composé de départ pour former un groupe pouvant se séparer, formant ainsi un composé de formule (1) ou (2) de la revendication 1, où X est H, ou
(b) dans lequel X est autre que H, où le composé qui est la source du groupe se séparant au couplage est un électrophile ou le composé de départ (3) contient du carbone entre l'atome de carbone du groupe carbonyle et R³ est substitué par le groupe se séparant au couplage.

8. Procédé selon la revendication 7 qui correspond à l'un des schémas de réaction suivants : 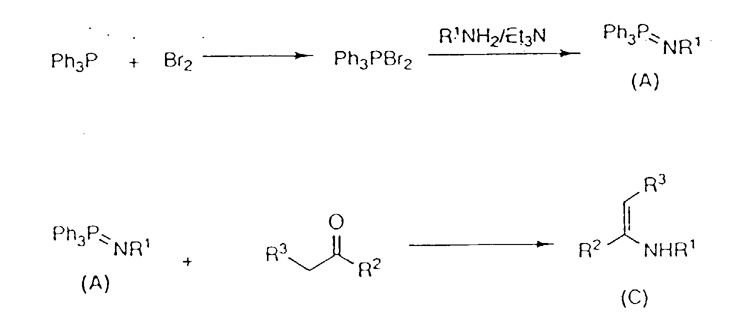 ou 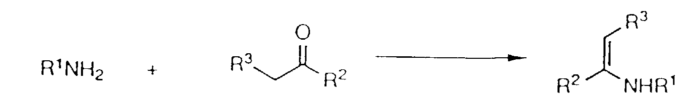 dans lequel R¹, R² et R³ sont tels que définis dans la revendication 1.
